# EUROPEAN PATENT APPLICATION

(11) **EP 4 310 553 A1**
(43) Date of publication of application: **24.01.2024**
(21) Application number: 22185746.9
(22) Date of filing: 19.07.2022
(51) Int. Cl.: G01T 1/10

(54) **DOSIMETER BASED ON NANO-SIZED PHOSPHOR PARTICLES**

(71) Applicant: AGFA NV, 2640 Mortsel (BE)
(72) Inventor: LEBLANS, Paul, 2640 Mortsel (BE); JUNG, Jürgen, 2640 Mortsel (BE); UYTTENDAELE, Carlo, 2640 Mortsel (BE); VANDENBROUCKE, Dirk, 2640 Mortsel (BE); HIMSCHOOT, Katleen, 2640 Mortsel (BE)
(74) Representative: Verbrugghe, Anne Marie L.

(57) **Abstract**

The present invention relates to a radiation dosimeter for accurately measuring the amount and spatial distribution of deposited dose by a source of radiation used in a radiation therapy treatment or treatment planning. The dosimeter comprises a coating layer that has a binder material, nano-phosphor particles that are homogeneously distributed in said binder material, and a microstructure added to said binder material that introduces a light-scattering effect.

## Description

### Technical Field

The present invention relates to a radiation dosimeter for accurately measuring the amount and spatial distribution of deposited dose by a source of radiation used in a radiation therapy treatment or treatment planning. The obtained measurements can be used in the context of quality assurance or quality control procedures that are applied to radiotherapy equipment within a radiotherapy department, or may be used in the context of patient specific quality assurance of a dose delivery treatment. In the latter case the radiation dosimeter may be applied in-situ during the one or more therapy sessions.

### Background of the invention

The field of radiation therapy has rapidly evolved over the past years. Whereas originally, patients were exposed to single fields of radiation on the body part to be treated, later evolving to multiple fields from various directions, now very sophisticated radiation generating machines are available, using linear accelerators which rotate around the patient while changing the shape of the beam (using a multi-leaf collimator to match the shape of the beam to the corresponding shape of the tumour), and even the intensity of the beam. This results in potentially very high treatment quality, where dose to the organs to be treated can be maximised, whereas dose to the neighbouring organs which need to be spared ("OAR", organs at risk) can be minimised.

However, for a radiation therapist or medical physicist in charge of maximising treatment quality and outcome, it is not intuitively possible to predict nor verify the effective delivered patient dose. Prediction of the dose in all 3 dimensions has to be done, and is implemented in what is called a 'Treatment Planning System' (TPS).

Most of these systems calculate dose on the basis of approximations and physics models. With increasing spatial modulation of the dose patterns administered, where some basic assumptions in most of these TPS such as local electronic equilibrium are not met anymore, there can be a discrepancy between predicted dose pattern and the real dose administered to the patient. Also, several other more trivial operator and machine errors are possible, and have happened in the past when treating patients, with dramatic consequences. Therefore, there is a rising demand for executing a pre-check of the dose administration, at least for complex irradiation patterns.

This is done before irradiating the patient, by irradiation of a dosimeter, placed at and around the location where the patient needs to be treated, in a medium that mimics the patient, often called "phantom", such as a cube or cylinder containing water, or blocks of "solid water", which is a mainly plastic based material. This solid water is easier to handle than real water and yet with a very similar response to radiation. According to this way, it is possible to verify if the TPS result is indeed going to be delivered to the patient, by comparing in each measurement point the dose actually measured with the TPS.

If the deviations are clinically relevant, this would then warrant the adaptation of the treatment plan and/or the machine settings until the desired actual dose pattern is delivered. Currently, a number of technologies are used, each however with significant drawbacks. It is the purpose of the present invention to overcome most of these drawbacks.

A radiation dosimeter has important requirements:
A. Dose accuracy. The absolute accuracy ideally desired is less than 1 per cent absolute error vs. true dose. To further maintain this accuracy over time, it is preferable to have a dosimeter that would need calibration only at long intervals, and/or would be very quick and easy to calibrate. A very important condition to reach accuracy is body equivalence. This is the first and foremost need for a dosimeter: The response should have the same energy dependence as the body tissue. In this context, it is important to note that body equivalence of a certain dosimeter technology is mainly a challenge for low-energy, scattered radiation. At high radiation photon energies (> 0.1 - 1 MeV), interaction of matter with radiation happens mainly through the Compton scattering mechanism, and all atoms from low to high Z atomic number have in a first approximation a similar response.

Since the beam energies used in radiation therapy are typically in the megavoltage range (a 6 MeV linac beam enters the body with an average energy in the order of 1.4 MeV; a Cobalt beam will have a similar energy of 1.173 -1.333 MeV, the Z value of the dosimeter material would not matter that much for this radiation.

However, there is a substantial amount of secondary, scattered radiation generated within the body during irradiation, with significantly lower energies, in the (1 - 1000) keV range. At these low radiation photon energies, interaction of matter with radiation happens for a substantial part through the photo-electric effect. This effect however is much more significant for high Z materials, and is very roughly proportional to Z³, multiplied by the density of the material.

Since the body consists mainly of water, with a so-called Z_{eff} of 7.22, it is important therefore to have the dosimeter respond also for this low-energy radiation similar to water. The most evident way is to try to approach the value of Z_{eff} of 7.22 in the closest possible way.

To be suitable for being placed inside a phantom as a stack of dosimeter plates in case of 3D radiation dosimetry, and hence behave as human tissue regarding the path of the radiation beam, the attenuation of the radiation beam by the radiation dosimeter should also be very similar to the human tissue.

Moreover, the response to radiation in function of incident beam angle should be also similar to body tissue as a necessary condition for body equivalence. Especially with the current rotating beams, where radiation can hit the patient and thus the dosimeter from all angles, it is extremely important that this condition is met.

Another necessary condition for dose accuracy is linearity of the dosimeter response - twice the amount of dose should result in a doubled dose number. Since many dosimeters are inherently non-linear, a calibration might be needed to convert to linear, accurate results.

B. Sufficient spatial resolution. With the ever increasing spatial detail of radiation dose patterns, a spatial resolution of dosimetric measurements should be able to reflect these modulations. At the current state of the art, a resolution in the order of 1 mm is highly desirable, and the trend to higher resolutions is present.

C. Sufficient dose range (dynamic range). Traditionally, irradiations are done in several sessions, typically 30 (called "fractions") of a moderate amount of locally applied radiation, e.g. 2 Gy. However, there is a trend to reduce the number of sessions, for clinical and also practical reasons. At this moment, doses per fraction can reach 20 Gy and higher. Therefore, it becomes mandatory to accurately measure doses up to this high dose level. It also becomes clear that at these doses, even small relative errors can be clinically important, more specifically for the OAR, and hence there is more need for QA effort by dosimetry before irradiating.

D. Dose rate independence. The dosimeter should provide an accurate dose reading, regardless of the rate (expressed in Gy/min) at which the dose was delivered.

E. Ease of use. The dosimeter should be easy to use, not requiring any special precautions, special handling etc.

F. Cost effectiveness. To have a low cost of ownership, the dosimeter should be reusable.

Computed Radiography (CR) has been proposed for use in radiation therapy dosimeters. CR systems inherently produce a linear dose-response over several decades of dose, but more importantly, CR systems are reusable and do not require a chemical processing step. Conventional CR systems for radiography will in general generate too many photo-electric electrons at low energies (corresponding with scattered radiation during therapy) as compared to body tissue, and are thus not suitable to represent patient dose as they will provide an over-estimate, especially in the areas where the primary radiation is weak (penumbra etc.). In 2005, A.J. Olch, Med. Phys., Vol. 32, No. 9, 2005 showed that BaFBrl:Eu2+-based computed radiography (CR) storage phosphor films (SPFs) had the potential to be used for two-dimensional megavoltage radiation therapy dosimetry. However, BaFBrl has a high Z number (Zeff = 49) which leads to a strong photon energy-dependence and consequently unacceptable measurement accuracy. The over-response to scattered radiation has been effectively reduced by using a thin lead foil on top of the plate (A.J. Olch, Med. Phys., Vol. 32, No. 9, 2005). However, this results in angle-dependence of the response, thus making the system unsuitable for any rotational therapy - which is however common practice at this moment. In radiotherapy treatment, cancer cells are killed by irradiation with X-rays, gamma-rays, electron beams and particle beams, the latter using hadrons such as protons, carbon ions and the like.

The goal of dosimetry in radiotherapy is to measure how much radiation energy is absorbed in an object and more specifically in body-tissue. For experimental purposes the body-tissue is often represented by water, that is then irradiated in an applied radiation field. Therefore, the signal produced by the dosimetry system should be proportional to the amount of energy absorbed in tissue, and this ideally for all radiation energies or energy spectra of applied dose.

In other words, the proportionality factor between the signal produced by the dosimeter and the energy absorbed in tissue should be the same for all X-ray energies, even if radiation is used having an unknown energy spectrum. Since most of the radiation sources used in radiotherapy do not have similar energy spectra or at least do not have identical energy spectra, this proportionality factor of the dosimeter should ideally be constant across the different radiation energies.

In most dosimetric applications, a 2D dosimeter is used, such as e.g. a classical silver halide (AgX) film, a GafChromic film, detector arrays or an EPID. The advantage of a film over electronic detector types, is that it can be inserted and placed in a phantom, attached to the skin of the patient or on a positioning device. Film dosimeters can be based on phosphors with different compositions and properties for use in systems based on optically stimulated luminescence (OSL), radio-luminescence (RL), thermos-luminescence (TL) or radio-photoluminescence (RPL). These different types of phosphors produce luminescent light based upon different principles of energy storage and excitation / stimulation.

In an OSL system, a so-called storage phosphor is used. During the irradiation, free electrons and holes are created, which are trapped in charge traps that are associated with crystal defects that are in the results of the phosphor composition and the manufacturing process. Stimulation of the phosphor with laser light can release the trapped electrons which then recombine with a dopant related trapped hole leading to a characteristic emission of photons that can be collected by a detector in a dedicated scanner, such as e.g. used in computed radiography for radiology applications. Moreover, this type of phosphor dosimeters is reusable.

In an RL-based system, a different kind of phosphor is used. During the irradiation, free charges are created that immediately recombine on a luminescent center, leading to direct emission of characteristic photons. These photons can be detected via a dedicated detection system, such as an optical camera, a TFT based detector such as in an EPID, or a photodiode or a PMT.

In TL-based systems, also a storage phosphor is used like in OSL, that stores the photo-charges in defect states. In TL the trapped photo-charges are released by heating the dosimeter, which leads to characteristic emission at certain temperatures. This emission is measured using a dedicated detection system.

In an RPL-based system, another type of storage phosphor is used, in which during irradiation new luminescent crystal defects are created. The number of the new luminescent defects in the phosphor is proportional to the accumulated absorbed radiation energy. This accumulated absorbed energy can be the result of a single irradiation or of multiple irradiations. The created luminescent centres are stable and do not fade over time. The number of created luminescent centres as a consequence of irradiation can be measured by exciting the centres and detecting their characteristic emission. Hence, the amount of stored energy can be measured with a dedicated detection system after each irradiation session at all time in between sessions or after finishing all therapy sessions.

The currently known OSL, TL, RL and RLP materials all contain higher Z elements and are, therefore, not body-equivalent.

In all of these systems, an optical signal can be measured, which after application of corrections leads to an absolute dose measurement, either in a single measurement point, a 2D dose profile or a volumetric dose profile representation of the actual dose deposited during the irradiation.

OSL-based dosimetry has been studied intensively so far, but either the prototype systems were based on phosphor screens used in radiology applications, or based on experimental phosphors which as such were not fulfilling all requirements of the application [Kandasamy S. et al, www.ojionline.org, DOI:10.4103/oji.oji_34_20, "Application of Computed Radiography in the Quality Assurance of Linear Accelerations in Radiotherapy."; Harold L H., Med. Phys. 34, (2007),103, DOI: 10.1118/1.2400617, "Dose response of BaFBrl : Eu 2 + storage phosphor plates exposed to megavoltage photon beams."].

High Z element containing phosphors have the disadvantage of absorbing a lot of energy when exposed to low energy photons, due to the photo-electric effect. During a therapy session, these lower energy photons are a results of scattering effects. This leads to the known over-response in all regions that are exposed to a high fraction of scattered radiation, like e.g. the out-of-field region that is not exposed to the direct beam.

The present invention tries to solve the problem of over-response of a phosphor based dosimeter in order to reduce the overestimated dose reading caused by scattered low-energy photons due to the photo-electric effect.

### Summary of invention

In current commercial phosphor based radiation dosimeters, the type of phosphor that is used is either a single crystal, is deposited by vapour deposition techniques, is obtained via solid state reaction or is obtained via a solvent based reaction. The resulting material is typically coated on a substrate as a high phosphor-to-binder layer. The crystal sizes obtained with these preparation techniques are micro-meter sizes or larger. In the context of this invention a coating layer is a phosphor-binder mixture that is coated as a layer on a substrate.

The present invention provides for nano-sized or sub-micrometer sized phosphor particles that can be coated on a substrate, and preparation methods therefore. The invention also provides for a dosimeter based on a substrate that is coated with a coating layer comprising such nano-sized or sub-micrometer sized phosphor particles.

The radiation energy absorbed in these small phosphor particles produces secondary electrons that can escape from the phosphor particle if it is small enough. The secondary electrons that escape from the phosphor particle deposit their energy in the surrounding medium if the packing of the phosphor particles is not too dense. The energy deposited outside of the phosphor particle will not contribute to the dose signal, thereby leading to a lower over-response for the low-energy radiation that is caused by the photo-electric effect. The applied principle is similar as for using metal nano-particles during radiotherapy session to increase the deposition in the cancerous tissue where gold particles are injected as described in; Briggs Samuel A., Hatter K, from "Gold Nanoparticles, ed Rahman M. and Asiri A.M., "Evolution of Gold Nanoparticles in Radiatioin Environments", DOI: 10.5772/intechopen.80366*;* Liu Y., et al, Theranostics. 2018; 8(7): 1824-1849. "Metal-based NanoEnhancers for Future Radiotherapy: Radiosensitizing and Synergistic Effects on Tumor Cells", DOI: 10.71501thno.22172*.*

However, in order to optimally gain from the application of nano-phosphors as an active component in the dosimeter of this invention, the nano-phosphors are mixed with a (preferably organic polymeric) binder material into a coating layer using a high binder-to-phosphor ratio, and this in order to increase the inter-particle distance of the nano-phosphor particles. In other words, the active nano-phosphor particles are diluted in the binder material, such that the nano-phosphor particles are sufficiently spatially separated from each other in order not to capture each other's secondary electrons.

Since the nano-phosphor particles (particle size in the nm-range) have lower sensitivity in comparison to the slightly larger micro-phosphors particles (particle size in the µm-range) of the same composition, it is advantageous for the clinical application to apply a relatively thick layer of the coating in order to ensure sufficient presence of active phosphor particles in the coating layer. In other words, a certain thickness of the coating layer is required in order for it to comprise sufficient phosphor material _{[L,P1]}at such high binder-to-phosphor ratio in order to achieve sufficient sensitivity.

As a consequence of the high binder-to-phosphor ratio, there is only limited light scattering introduced by the nanometric phosphor particles at low phosphor concentration, resulting in a translucent coating layer, which has an optical free path comparable to the distance between the phosphor particles. In such a translucent layer, any optical photons (used for stimulation or caused by emission) will therefore diverge or spread over a relatively large area within the coating due to light piping in the coating layer. And the distance over which this light piping occurs increases with the thickness of the layer. Consequently, the sharpness of the measurable dose profile produced by the coating layer is lowered, causing an undesirable effect of a low gamma performance of the dosimetry system.

To overcome this problem of low gamma (low sharpness), the dosimeter of the invention comprises a combination of nano-phosphor particles coated in a thick polymer layer in which the polymeric layer contains a micro-structure in its volume. The micro-structure is meant to introduce additional scattering of the optical radiation. An example of the realization of such a volumetric micro-structure is given by the inclusion of small spacer particles into the thick polymer layer and a precise control of the volume ratios of polymeric binder (polymer matrix) to spacer particles.

The spacer particles that are used to introduce enhanced light scattering should ideally consist of material with body equivalent Z value, such as for instance polymeric beads (atomic species include therefore C, H, O, N and S). Glass beads are a possible inorganic alternative to introduce the required scattering effect, but have -due to their composition- a larger effective Z-value compared to organic alternatives. Other examples of inorganic alternatives with relatively low effective Z-value and refractive index (RI) higher than that of the binder material comprise AIN (RI = 2,03); BeO (RI = 1,7); MgO (RI = 1,71); ALN-Al₂N₃ (RI = 1,79) and Si₂N₃ (RI = 2,07).

The materials that are selected as spacer particles should be transparent in the spectral ranges of the excitation and/or emission light. The spectral ranges involved in the dose detection process by the dosimeter depends on the type of luminescence that the phosphor nano-particle exhibits: which may be one of TL, RL, RPL or OSL. In case of insufficient transparency, the sensitivity of the dosimeter would drop.

A difference between the refractive index of the spacer particles and the binder material (polymer matrix) is advantageous for the dosimetry application, but is not strictly required. Typical polymers that would be used as binder material have refractive indexes between 1,45 and 1,55 (at λ = 589 nm), whereas the refractive index of air is 1,00. Typical spacer particles have refractive indexes between 1,40 and 1,60. In practice, the difference between the refractive indexes of the binder material and the spacer particles may not exceed 0,2, which introduces some optical scattering. However, the corresponding scattering efficiency introduced this way is typically not high enough to create a level of light diffusion that is needed to overcome the low sharpness of the detector layer due to lateral light piping.

The main optical scattering effect obtained from the introduction of the spacer particles is however not the above mentioned difference between the refractive indexes of the particles and the binder material itself, but rather the difference between the refractive indexes of the particles and air voids. The air voids are indirectly introduced in the coating layer by the spacer particles by the control of the amount of binder during the production process of the dosimeter coating layer. This represents a volumetric micro structuration of the layer, in which the difference between the refractive indexes of the particles and the air voids is quite large (about 0,50) and therefore causes a great scattering effect.

The air voids are created by selecting a suitable volumetric ratio of the binder material (polymer matrix), the spacer particles and the phosphor particles. In order to introduce air voids, the film formation of the polymeric binder across the spacer particles must be incomplete, meaning that the voids between the spacer particles should not be entirely sealed by the binder material, leaving the air voids in existence. To achieve this, the spacer particles should represent the majority of the total volume of the dosimeter material.

By mechanically mixing the above composition, a percolating structure comprising a random stack of spacer particles with interstitial air voids is obtained. There should be just enough polymeric binder used to "glue" the particles together, but without filling the entire interstitial volume between the percolated spacer particles. The volumetric fraction of phosphor particles is negligible, since its particle size and its particle size distribution (PSD) is much smaller in comparison with the particle sizes of the spacer particles, so that their contribution to the percolating structure is not decisive. The phosphor particles are more likely fully embedded in polymeric binder. Therefore, their contribution to optical scattering is limited, which has the advantage that the sensitivity of the detector coating can be adjusted by control of the amount of phosphor particles without affecting the overall optical transport in the scattering layer. The air voids (making up the interstitial volume) act as very efficient optical scattering centres.

The size and number distribution of the air voids cannot be predicted, but must be fine-tuned via the coating formulation that has to be optimized through experimental design. The ratios of binder-to-spacer volume, the particle size distribution of the spacer particles, the particle wettability and the film forming properties are important factors influencing the final result.

Other mechanisms besides the choice of the formulation for introducing the air voids in the polymeric binder may be considered including for instance: intense stirring of a viscous coating formulation ("foaming") or in-situ gas formation. The latter process may be otherwise known from the production of a so-called vesicular film. Both processes are however more difficult to control. When applying the in-situ gas formation method, the amount of gas volume and the particle size distribution of the gas bubbles may be difficult to control. This poor control could result in an unacceptable variability of optical scattering that would have a negative impact on the sensitivity, homogeneity and/or sharpness of the dosimetry detector.

The use of porous silica as a material for the spacer particles has been investigated, but has typically resulted in rather low scattering efficiency. Furthermore, silica is less preferred due to its high effective atomic number.

As explained above, the spacer particles introduce small air voids in the layer which lead to a high scattering of optical photons in the vicinity of their incidence or origin. In the case of sufficiently strong light scattering by a random volumetric micro structure introducing refractive index inhomogeneity, the optical mean free path will become comparable to or smaller than the thickness of the detector layer. Due to this high scattering condition, the effect of light piping is essentially reduced and the stimulation or emission light is better confined in the close proximity of where it is originating in the layer. This latter effect has a positive impact on the sharpness of the dosimetry system. For the present dosimeter application, the level of sharpness introduced by isotropic scattering from a random volumetric micro-structure of the refractive index seems sufficient. However, for higher level of sharpness an oriented micro-structure of the detector would be preferred, e.g. needles, tube or similar structures that are oriented perpendicular to the coating layer.

The spacer particles in the layer should ideally not contain high Z elements to preserve a good body equivalence of the material, and may be organic or inorganic in nature. The use of these sub-micrometer to nanometer phosphors in combination with the spacer particles that introduce air voids is leading to more accurate dose profiles when used to measure the dose in regions where the contribution of secondary radiation is important and guarantees a good gamma performance for the system.

The combination of phosphor nano-particles with a volumetric micro-structure introducing additional light scattering will reduce the importance of correction factors for low-energy over-response and field size dependence, which make the dosimetric system less accurate and reliable.

The improvement realized by using small phosphor particles combined with a low packing density and a volumetric micro-structure introducing additional light scattering is independent of the dosimetric technology used. It will lead to an improvement of TL, RL, RPL and OSL-based systems.

### Description of embodiments

In the following detailed description, reference is made in sufficient detail allowing those skilled in the art to practice the embodiments explained below.

The nano-phosphor particles according the present invention can be any suitable phosphor which absorbs radiation and charged particle radiation, and then either temporarily stores the energy of the absorbed radiation, or converts it into visible light. As explained, any phosphor type that exhibits any type of luminescence is suitable: which may be one of TL, RL, RPL or OSL.

The absorbed radiation energy is measured as luminescence radiation, as visible light and/or ultraviolet radiation, by stimulating the stimulable phosphor by means of radiation in the infrared or visible range. The stimulable phosphors which are applicable in the invention are preferably particles, having a particle size d₅₀ between 10 and 600 nm, more preferably between 50 and 500 nm, most preferably between 100 and 300 nm (less than 1µm for d₉₉).

Suitable stimulable phosphors comprise Bariumfluorohalide phosphors as disclosed in, e.g., US P 4.239.968, DE OS 2 928 245, US-P 4 261 854, US-P 4539 138, US P 4.512.911, EP 0 029 963, US-P 4 336 154, US-P 5 077 144, US-P 4 948 696, Japanese Patent Provisional Publication N°. 55(1980)-12143, Japanese Patent Provisional Publication N°. 56(1981)-116777, Japanese Patent Provisional Publication N•. 57(1982)-23675, US-P 5 089170, US-P 4 532 071, DE OS 3 304 216, EP 0 142 734, EP 0 144 772, US-P 4 587 036, US-P 4 608 190, and EP 0 295 522. BaFBr doped with Eu (BaFBr:Eu) and BaFBrl doped with Eu (BaFBrl:Eu) are preferably suitable.

Ba₁₋ₓSrₓF_{2-a-b}BrₐX_{b}: zA, wherein X is at least one member selected from the group consisting of CI and I; x is in the range 0.10 ≦ x ≦ 0.55 ; a is in the range 0.70 ≦ a ≦ 0.96; b is in the range 0 ≦ b < 0.15 ; z is in the range 10⁻⁷ < z ≦ 0.15, and A is Eu²⁺ or Eu²⁺ together with one or more of the codopants selected from the group consisting of Eu³⁺, Y, Tb, Ce, Tm, Dy, Pr, Ho, Nd, Yb, Er, La, Gd and Lu, and wherein fluorine is present stoichiometrically in said phosphor in a larger atom% than bromine taken alone or bromine combined with chlorine and/or iodine, as disclosed in EP 345 903. BaSrFBr is preferably suitable.

Alkali metal phosphors comprising earth alkali metals as disclosed in e.g. US-P 5,028,509 and EP 0 252 991. Alkalimetal phosphors as e.g. CsBr:Eu, RbBr:TI and KCI:Eu are preferably suitable.

Halosilicate phosphors as disclosed in, e.g. EP 304 121, EP 382 295 and EP 522 619.

The nano-phosphor particles are to be dispersed into a binder to form a nano-phosphor containing layer.

Preferred binders according to the present invention comprise organic polymers such as polyethylene glycol acrylate, acrylic acid, butenoic acid, propenoic acid, urethane acrylate, hexanediol diacrylate, copolyester tetracrylate, methylated melamine, ethyl acetate, methyl methacrylate, cellulose acetate butyrate, polyalkyl (meth)acrylates, polyvinyl-n-butyral, poly(vinylacetate-co-vinylchloride), poly(acrylonitrile-co-butadiene-co-styrene), poly(vinyl chloride-co-vinyl acetate-co-vinylalcohol), poly(butyl acrylate), poly(ethyl acrylate), poly(methacrylic acid), poly(vinyl butyral), polyurethane, trimellitic acid, butenedioic anhydride, phthalic anhydride, polyisoprene and/or a mixture thereof. The binder may also be a silicone-based binder, or thermally cross-linked epoxies or polyurethanes. Preferably, the binder comprises one or more styrene- hydrogenated diene block copolymers, having a saturated rubber block from polybutadiene or polyisoprene, as rubbery and/or elastomeric polymers. Most preferred binders are copolymers of vinyl isobutyl ether and n-butyl acrylate.

The binder, prior to the dispersing of the stimulable phosphor particles is preferably solubilised into an appropriate solvent. Appropriate solvents comprise for example lower alcohols such as methanol, ethanol, n-propanol and n-butanol; chlorinated hydrocarbons such as methylene chloride and ethylene chloride; ketones such as acetone, methyl ethyl ketone and methyl isobutyl ketone; esters of lower alcohols with lower aliphatic acids such as methyl acetate, ethyl acetate, propyl acetate and butyl acetate; toluene; ethers such as dioxane, ethylene glycol monoethylether and ethylene glycol monoethylether; and mixtures of the above-mentioned compounds.

To the solution of the binder, stimulable nano-phosphor particles can then be added. The addition is preferably performed under stirring and commonly known dispersion techniques can be used to obtain a finely divided dispersion that can be mixed in its turn with the spacer particles.

In a preferred embodiment will the volume ratio between the spacer particles and the dispersion (the nano-particles suspended in the binder) have to be chosen such that the dispersion does not occupy all air voids between the irregularly shaped spacer particles. The ratio has to be chosen such that air voids deliberately take up part of the volume of the coating layer, and that the dispersion does not completely saturate the spacer particle matrix. In a preferred embodiment, the air voids take up to 5% to 35% of the volume of the coating layer.

In an alternative embodiment, the volume ratio between the spacer particles and the dispersion will be chosen such that the dispersion completely fills up the air voids between spacer particles such that no air voids are present. In this embodiment will it be preferable to apply spacer particles of which the diffraction index is preferably different from the diffraction index of the binder material.

In this embodiment, glass spheres, glass particles, or spheres from different materials may be used. The spheres may be solid or could comprise a gas such as air in order to enhance the difference between the refractive indexes at the material interfaces.

The coating dispersion may contain a dispersing agent to assist the dispersibility of the phosphor particles therein, and also contain a variety of additives such as a plasticizer for increasing the bonding between the binder and the phosphor particles in the phosphor layer. Examples of the dispersing agent comprise phthalic acid, stearic acid, caproic acid and a hydrophobic surface active agent. Examples of the plasticizer comprise phosphates such as triphenyl phosphate, tricresyl phosphate and diphenyl phosphate; phthalates; glycolates and polyesters of polyethylene with aliphatic dicarboxylic acids.

While the coating layer may be a self-supported layer, an optional substrate may provide more physical support when the coating layer is coated on a side of a substrate. The substrate for the phosphor containing layer according to the invention, is preferably one which has a low radiation (e.g. X-rays, gamma-rays,...) absorptivity. The low absorptivity for X-rays, gamma rays, charged particle radiation is required in order to achieve a water-equivalent behaviour of the radiation dosimeter, more specifically if used in a stack of dosimeters. It can be either rigid or flexible, such as an aluminium plate, an aluminium foil, a film of polyethylene terephthalate (PET), polyethylene naphthalate (PEN), polyimide (PI), polyethersulphone (PES), a metal foil, a carbon fibre reinforced plastic (CFRP) sheet, glass, flexible glass, triacetate and a combination thereof or laminates thereof. Preferred materials for the substrate to be used in the invention comprise PET, PEN and glass.

The coating dispersion containing the phosphor particles, the binder and the spacer particles, can be applied evenly to the surface of a substrate to form a layer of the coating dispersion.

The coating can be carried out by a conventional method such as a method using a doctor blade, a roll coater or a knife coater. Subsequent to the coating, the formed phosphor containing layer can be dried at ambient temperature or at increased temperature.

Coating is an economically efficient technique of application of one or more layers onto a substrate. By means of coating techniques, the phosphor containing layer can be applied together with intermediate layers and / or top layers, but also adhesion improving layers etc. Flexible substrates are particularly suitable for a continuous coating process. Moreover, flexible substrates can be available as rolls and they can be wound and un-wound in the production process of coating and drying or curing.

The thickness of the phosphor containing layer is within the range of 10 µm to 1 mm, preferably 20 µm to 500 µm, more preferably 50 µm to 250 µm.

## Claims

1. Dosimeter for radiotherapy applications, comprising
a coating layer, comprising;
- a binder material,
- nano-phosphor particles that are homogeneously distributed in said binder material, and
- a micro-structure added to said binder material that introduces a light-scattering effect.

2. Dosimeter of claim 1 wherein said micro-structure comprises spacer particles mixed with said binder material in such a volumetric ratio that a percolating structure is obtained wherein air voids are provided between said mixture of spacer particles and binder material.

3. Dosimeter of claim 1 wherein said micro-structure comprises inorganic particles with low effective atomic number (Z value) and a refractive index (RI) value that is different from that of said binder material.

4. Dosimeter of Claim 1, comprising a substrate supporting said coating layer on a side of said substrate.

5. Dosimeter according to Claim 2, wherein said spacer particles consist of material with substantially body equivalent Z value.

6. Dosimeter according to Claim 1, wherein said nano-phosphor particles have a particle size d₅₀ between 10 nm and 600 nm.

7. Dosimeter according to Claim 6, wherein said nano-phosphor particles have a particle size d₅₀ between 50 nm and 500 nm.

8. Dosimeter according to Claim 7, wherein said nano-phosphor particles have a particle size d₅₀ between 100 nm and 300 nm .

9. Dosimeter according to Claim 2, wherein said spacer particles have a particle size d₅₀ between 0,5 µm and 20 µm, preferably 1 µm and 10 µm, most preferably between 2 µm and 6 µm.

10. Dosimeter according to Claim 2, wherein said spacer particles are arranged to provide needles, tube or similar structures that are oriented perpendicular to the coating layer.

11. Dosimeter according to Claim 2, wherein said spacer particles consist of glass beads.

12. Dosimeter according to Claim 1, wherein said binder material comprises an organic polymer matrix.

13. Dosimeter according to Claim 1, wherein Z_{eff} of said coating layer approximates the Z_{eff} of water.

14. Dosimeter according to Claim 1, wherein said nano-phosphor particles exhibit one of the following luminescence effects: optically stimulated luminescence (OSL), radio luminescence (RL), thermoluminescence (TL) or radio photo luminescence (RPL).

15. Dosimeter according to claim 1, wherein said coating layer has a thickness within the range of 10 µm to 1 mm.

16. Dosimeter according to claim 1, wherein said coating layer has a thickness within the range of 20 µm to 500µm.

17. Dosimeter according to claim 1, wherein said coating layer has a thickness within the range of 50µm to 250µm.

18. Dosimeter according to claim 2, wherein the air voids take up 5% to 35% of the volume of the coating layer.
